# FASCICULE DE BREVET EUROPEEN

(11) **EP 1 503 803 B1**
(45) Date de publication et mention de la délivrance du brevet: **30.07.2008**
(21) Numéro de dépôt: 03752788.4
(22) Date de dépôt: 07.05.2003
(51) Int. Cl.: A61K 49/04, A61K 31/045, A61K 47/38, A61K 9/08

(54) **COMPOSITION PHARMACEUTIQUE INJECTABLE COMPRENANT DE L'ÉTHANOL POUR LE TRAITEMENT DES HERNIES DISCALES**
INJIZIEBARE PHARMAZEUTISCHE ZUSAMMENSETZUNG ENTHALTEND ETHANOL ZUR BEHANDLUNG VON BANDSCHEIBENVORFÄLLEN
INJECTABLE PHARMACEUTICAL COMPOSITION COMPRISING ETHANOL FOR TREATING HERNIATED INVERTEBRAL DISCS

(30) Priorité: 07.05.2002 FR 0205819
(43) Date de publication de la demande: 09.02.2005
(73) Titulaire: Theron, Jacques, 14123 Fleury sur Orne (FR)
(72) Inventeur: THERON, Jacques, F-14123 Fleury Sur Orne (FR)
(74) Mandataire: Catherine, Alain
(86) Numéro de dépôt international: PCT/FR2003/001412
(87) Numéro de publication internationale: WO 2003/097108

(56) Documents cités:
- WO-A-00/71064
- WO-A-00/71170

## Description

L'invention concerne une préparation médicamenteuse injectable visqueuse contenant notamment de l'éthanol de l'ethylcellulose et un composé opaque aux rayons X.
Elle s'applique plus particulièrement, mais non exclusivement, au traitement des hernies discales mais également aux procédures interventionnelles ayant démontré l'efficacité de l'éthanol pur : traitement des tumeurs hépato-cellulaires ou encore des ostéomes ostéoïdes, des kystes des reins et des angiomes artérioveineux.

Les hernies discales sont les principales causes des douleurs lombaires et des sciatiques. Elles sont le plus souvent relatives à de multiples facteurs ergonomiques et anatomiques : mauvaises positions, faiblesse des muscles abdominaux et paraspinaux.

Les thérapies conservatrices et chirurgicales sont les traitements choisis dans la plupart des cas. Cependant, chez certains patients sélectionnés avec des critères cliniques et radiologiques bien définis, un traitement percutané peut être proposé.

L'efficacité du traitement percutané des hernies discales lombaires par injection d'une enzyme, la chymopapaïne, (nucléolyse : modification enzymatique d'un disque intervertébral) a été bien démontrée. Cependant, des patients avec des antécédents allergiques ou déjà traités par nucléolyse, ne peuvent pas profiter de cette procédure.

Au regard des actions nécrosantes de l'éthanol sur les tissus biologiques, l'alcool absolu (éthanol anhydre) a été utilisé comme un agent thérapeutique efficace dans de nombreuses procédures d'intervention : sclérose de ganglions et de nerfs, ablation de tumeurs hépatiques, de tumeurs rénales, traitement préopératoire de tumeurs vertébrales, malformations artérioveineuses périphériques, viscérales et du cerveau.

Une équipe a récemment pensé que ces propriétés thérapeutiques pouvaient être utilisées dans des pathologies lombaires. Elle a traité des hernies discales lombaires avec des injections intradiscales d'alcool absolu avec des résultats très encourageants. Cette procédure percutanée présente certains avantages :
- pas de complication allergique,
- pas de complications septiques locales,
- pas de douleur importante après traitement,
- pas de rétrécissement de l'espace interdiscal,
- pas de complications inflammatoires,
- un temps très court de récupération clinique.
Cependant, l'éthanol, de par ses propriétés, peut diffuser loin de sa cible et nécroser des cellules saines. C'est la raison pour laquelle, l'équipe utilisant l'éthanol pur a contre-indiqué son usage dans les hernies discales avec fuite épidurale visualisée à la discographie et dans les cas de hernies discales cervicales.

De surcroît, dans le traitement de tumeurs hépato-cellulaires, l'alcool peut provoquer des thromboses de la veine porte par diffusion du produit dans le système veineux.

La demanderesse a, entre autres, pour objectif d'éviter les effets secondaires de l'éthanol dans cette pathologie, à savoir la nécrose des tissus sains en raison d'une diffusion loin de la cible tout en renforçant son efficacité.

A cet effet, elle a testé plusieurs substances épaississantes et elle propose une préparation médicamenteuse injectable comprenant au moins un composé pour donner une préparation visqueuse, de l'éthanol et au moins un composé rendant ladite préparation opaque aux rayons X de manière à contrôler son administration et son action.

Avantageusement, la viscosité permet de limiter la diffusion de la préparation à une zone définie et de renforcer son effet thérapeutique.

Ainsi, cette préparation présente le double avantage de proposer un principe actif dont la diffusion est limitée et un marqueur qui permet de mieux suivre l'injection durant l'intervention du thérapeute et surtout de contrôler après le traitement la situation exacte du produit injecté à l'aide d'un scanner.

Les composés utilisés pour rendre la préparation opaque aux rayons X pourront être des composés inertes tels qu'un oxyde de tungstène ou un oxyde de tantale.

Ces composés pourront être incorporés, par exemple sous forme de poudre, soit à la fin de la fabrication de la préparation, soit juste avant l'intervention.

Des compositions injectables comprenant un solvent (tel que l'éthanol), un polymère (tels que les acétates de cellulose) et un agent de contraste biocompatible ont été décrites dans WO00/71064A et WO 00/71170A.

L'éthylcellulose a été choisi comme composé (excipient) pour obtenir la viscosité souhaitée. Ce choix repose sur plusieurs critères :
- son hydrophilie, en raison du caractère injectable de la préparation,
- son pouvoir épaississant qui devait être suffisamment important pour augmenter, même présent en faible quantité, la viscosité du mélange,
- les dérivés de la cellulose sont hydrolysables in vitro et dispensent ainsi d'une résection chirurgicale de la zone soignée,
- une présentation sous forme de poudre et non de liquide afin de ne pas diluer l'éthanol,
- une certaine solubilité dans l'éthanol de manière à obtenir une préparation homogène,
- des effets toxiques locaux et/ou systémiques réduits au minimum voire inexistants pour ne pas compromettre la tolérance de la préparation.

Par conséquent, la préparation selon l'invention répond aux critères essentiels requis qui sont la sûreté d'emploi dans toutes les phases du processus, l'aptitude à créer une sclérose stable et sélective.

Un mode d'exécution de l'invention sera décrit ci-après; à titre d'exemple non limitatif :
La figure 1 représente la mesure de la viscosité exprimée en unité pharmaceutique en fonction de la concentration exprimée en pourcentage du poids d'éthylcellulose par rapport au poids total ;
La figure 2 représente la mesure de la viscosité exprimée en unité pharmaceutique en fonction de la température exprimée en degré Celsius.

### Choix des excipients entrant dans la préparation

Six produits inscrits à la Pharmacopée européenne 3^{ème} édition (1999) et répondants aux critères mentionnés ci-dessus ont donc été choisis pour être évalués comme excipient (Tableau I). Pour chacun d'entre eux, la solubilité à chaud et/ou à froid dans l'éthanol a été étudiée de même que la compatibilité physico-chimique des substances mises en contact (par appréciation visuelle) et enfin la viscosité approximative du mélange.

**Tableau I : Produits testés pour la fabrication du gel**

| Produits | Références commerciales | Fournisseurs |
|---|---|---|
| Hydroxycellulose | Klucel MF EP® | Aldrich |
| | Klucel HF EP® | |
| Ethylcellulose | Aqualon 100 NF ® | Hercules |
| Polysorbate | Montanox 80® | Seppic |
| Silice colloïdale | Aérosil R972® | Degussa France |
| | Aérosil R200® | |
| Carboxypolyméthylène | Carbopol 940® | Gattefosse |
| | Carbopol 934® | |
| Polyéthylèneglycol | Lutrol E4000® | BASF France |
| | Lutrol E6000® | |

Les résultats des différents essais sont résumés dans le tableau II. Tous les produits testés étaient solubles à chaud dans l'éthanol. Seule l'éthylcellulose l'était plus ou moins également à froid. De plus, la compatibilité physicochimique du mélange (absence de précipitation) était bonne et la viscosité satisfaisante. C'est pour ces raisons que l'éthylcellulose a paru le produit le plus adapté.

**Tableau II : Caractéristiques des produits testés**

| Produits | Solubilité dans l'éthanol | | Examen visuel |
|---|---|---|---|
| | A froid | A chaud | |
| Hydroxypropylcellulose | - | + | Après refroidissement, non homogène |
| | - | + | |
| Ethylcellulose | ± | + | Gel limpide |
| Polysorbate | - | + | Viscosité très faible |
| Silice colloïdale Silice colloïdale | - | + | La viscosité n'apparaît qu'après quelques jours |
| | - | + | |
| Carboxypolyméthylène | - | + | Précipite après neutralisation |
| | - | + | |
| Polyéthylèneglycol | - | + | Précipité à froid |
| | - | + | |

### Fabrication de la préparation

Plusieurs préparations à différentes concentrations en éthylcellulose ont été effectuées par dissolution respectivement de 0,15 ; 0,45 et 0,75 g dans 15 mL d'éthanol d'une pureté de 70 à 99 % en volume et de préférence de 95 % (d = 0,8) soit 1,22 ; 3,61 et 5,88 % en poids du poids total de la préparation.
La préparation dont la concentration en éthylcellulose est la plus élevée a été choisie. Une perte de 2,5 % au moment , de la répartition en flacons a été considéré soit pour quarante flacons, des proportions de 205 mL d'alcool à 95° (% volume) et 10,25 g d'éthylcellulose.
Plus généralement, l'éthylcellulose est présent en une concentration variant de 0,5 à 15 %, de préférence 5,88 % en poids du poids total de la préparation.

Conformément aux Bonnes Pratiques de Fabrication (1998), la préparation a été réalisée en trois étapes : préparation du gel, répartition aseptique et stérilisation du produit dans le conditionnement final. Dans un premier temps, l'excipient (éthylcellulose) a été mélangé à l'éthanol dans un ballon à col rodé stérile, à chaud, par agitation magnétique et à reflux jusqu'à dissolution complète. Le tout a été laissé sous agitation et avec le reflux pendant quinze minutes, puis sous agitation jusqu'à refroidissement afin de permettre la recondensation de l'alcool dans le ballon. Le reconditionnement s'est fait ensuite sous hotte à flux laminaire horizontal dans des flacons sertis stériles de 5 mL (bioblock 42065). Enfin, comme le préconise la Pharmacopée européenne, les flacons ont été stérilisés à l'autoclave par vapeur saturée, à 121°C pendant vingt minutes.

La dernière étape de fabrication de la préparation, l'ajout d'une poudre de composé opacifiant tel que l'oxyde de tantale ou l'oxyde de tungstène, se fait en proportion variable pour obtenir une bonne opacité. Cet ajout peut avoir lieu soit à la fin de la fabrication avant le reconditionnement, soit juste avant de pratiquer l'intervention.

Par ailleurs, ces composés étant inertes et utilisés en très faible quantité, ils ne modifient pas significativement les résultats des contrôles présentés ci-dessous et effectués sur la préparation avant leur ajout.

### Contrôles effectués

La conformité de la préparation a été vérifiée par un essai de stérilité, des contrôles chimiques et physicochimiques.

Selon les recommandations de la Pharmacopée européenne, la présence éventuelle de contaminants a été recherchée par ensemencement de 4 mL de la préparation dans 250 mL de bouillon tryticase-soja pour les germes aérobies, au thioglycolate pour les germes anaérobies et Sabouraud pour les levures.
Les résultats de l'essai de stérilité ont confirmé l'absence de contaminants dans la préparation.

La teneur en alcool a été déterminée après dilution de l'échantillon et incorporation de l'étalon interne, le propanol-1, par chromatographie gazeuse avec détection par ionisation de flamme. La séparation est obtenue sur une colonne Porapak Q (80-100 « mesh » (maille), longueur 3 m) avec l'azote comme gaz vecteur (1,2 bar) sur un appareil Delsi DN200.
Le dosage de l'alcool a donné une valeur de 802 g.L⁻¹.

Le dosage spécifique de l'adjuvant de viscosité n'a pas été réalisé, mais la concentration a été estimée par la méthode des résidus secs, opération qui consiste à évaporer l'éthanol dans une cuve thermostatée à 110°C jusqu'à poids constant de l'échantillon.
La méthode des résidus secs a permis de corréler la concentration théorique en éthylcellulose à celle mesurée expérimentalement soit 5,88 % en poids du poids total de l' échantillon.

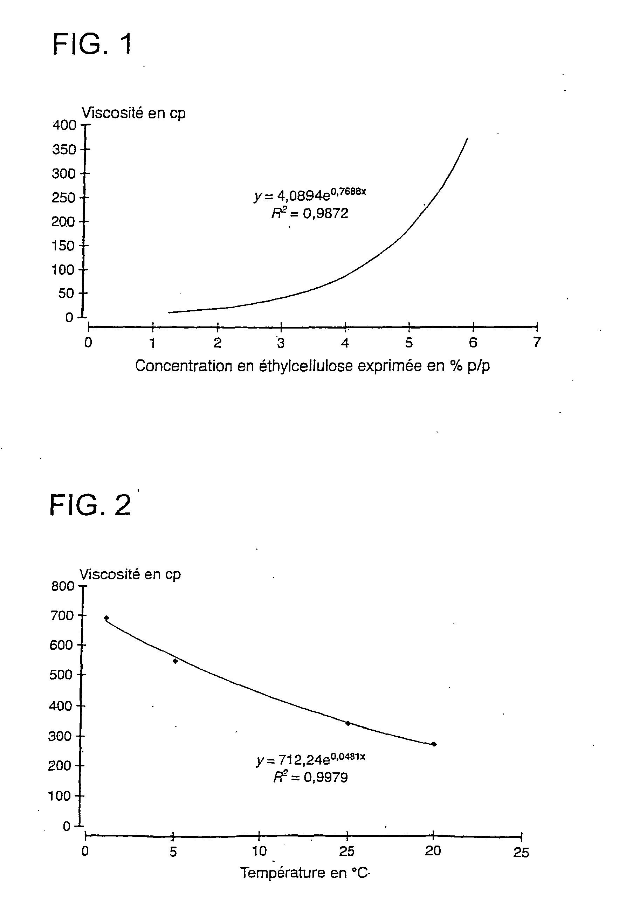

La viscosité de la préparation a été mesurée à l'aide d'un viscosimètre à capillaire dit de Baumé (Prolabo). Plusieurs séries de mesure ont été effectuées à différentes températures et à différentes concentrations en épaississant la préparation.
Les mesures de viscosité ont montré que, à température constante, la viscosité de la préparation augmentait de manière exponentielle avec la teneur en éthylcellulose (Figure 1). En revanche, elle diminuait, toujours de façon exponentielle, lorsque la température augmentait (Figure 2).

Enfin l'étude de la stabilité physicochimique a été réalisée par une analyse dans le temps de l'évolution des paramètres qui définissent la préparation c'est-à-dire la viscosité, la teneur en éthanol et en agent de viscosité. Les mesures ont été répétées à un jour (J1), huit jours (J8), quinze jours (J15), trente jours (J30).
Les résultats sont consignés dans le tableau III. Les coefficients de variation inférieurs à 3% prouvent la stabilité du mélange jusqu'à J30, ce qui permettra de déterminer une date de péremption pour la préparation.

**Tableau III : Paramètres physicochimiques en fonction du temps**

| Date | C_{éthanol} (g.L⁻¹) | V_{éch} (ML) | P_{éch} (g) | d_{éch} (g.mL⁻¹) | Résidu sec RS (g) | Rapport RS/P_{éch} (%) | Viscosité (cp)* |
|---|---|---|---|---|---|---|---|
| J1 | 784 | 2 | 1,650 | 0,825 | 0,101 | 6,10 | 320 |
| J8 | 821 | 2 | 1,720 | 0,850 | 0,103 | 5,97 | 339,5 |
| J15 | 783 | 2 | 1,610 | 0,800 | 0,097 | 6,00 | - |
| J30 | 820 | 2 | 1,670 | 0,830 | 0,099 | 5,92 | 332 |
| Moyenne | 802 | - | 1,663 | 0,826 | 0,100 | 5,998 | 330,5 |
| Ecart-type | 21,370 | - | 0,046 | 0,021 | 0,002 | 0,076 | 9,836 |
| Coefficient de variation | 2,665 | - | 2,751 | 2,489 | 2,458 | 1,265 | 2,976 |

| | | | | | | | |
|---|---|---|---|---|---|---|---|
| * cp : unité pharmaceutique | | | | | | | |

Lors de son injection dans un disque lombaire, la préparation a permis de diminuer la pression intra-discale et ainsi de diminuer les douleurs lombaires dues aux hernies discales.

## Revendications

1. Préparation médicamenteuse injectable, **caractérisée en ce qu'**elle comprend au moins de l'éthylcellulose soluble à froid dans, de l'éthanol et au moins un composé rendant ladite préparation opaque aux rayons X.

2. Préparation selon la revendication 1, **caractérisée en ce que** l'éthylcellulose est présent en une concentration variant de 0,5 à 15%, de préférence 5,88% en poids total de la préparation.

3. Préparation selon la revendication 1, **caractérisée en ce que** ledit composé rendant ladite préparation opaque aux rayons X est un oxyde inerte.

4. Préparation selon la revendication 3, **caractérisée en ce que** ledit composé rendant ladite préparation opaque aux rayons X est un oxyde de tantale.

5. Préparation selon la revendication 3, **caractérisée en ce que** ledit composé rendant ladite préparation opaque aux rayons X est un oxyde de tungstène.

6. Préparation selon la revendication 1, **caractérisée en ce que** ledit éthanol a une pureté qui varie de 70 à 99% en volume, de préférence 95%.

## Claims

1. Medicinal injectable preparation, **characterised in that** it comprises at least ethycellulose soluble in cold ethanol, ethanol and at least one compound to make the said preparation opaque to X-rays.

2. Preparation according to claim 1, **characterised in that** ethylcellulose is present at a concentration ranging from 0.5 to 15% preferably 5.88%, by weight of the total weight of the preparation.

3. Preparation according to claim 1, **characterised in that** the said compound making the said preparation opaque to X-rays is an inert compound.

4. Preparation according to claim 3, **characterised in that** the said compound making the said preparation opaque to X-rays is tantalum oxide.

5. Preparation according to claim 3, **characterised in that** the said compound making the said preparation opaque to X-rays is tungsten oxide.

6. Preparation according to claim 1, **characterised in that** the said ethanol has a purity of 70 to 99% by volume, preferably 95%.

## Patentansprüche

1. Injizierbares medikamentöses Präparat, **dadurch gekennzeichnet, dass** es wenigstens Ethylcellulose, die in kaltem Ethanol löslich ist, Ethanol und wenigstens eine Verbindung, die das Präparat röntgenundurchlässig macht, umfasst.

2. Präparat gemäß Anspruch 1, **dadurch gekennzeichnet, dass** die Ethylcellulose in einer Konzentration vorhanden ist, die von 0,5 bis 15% variiert und vorzugsweise 5,88% des Gesamtgewichts des Präparats beträgt.

3. Präparat gemäß Anspruch 1, **dadurch gekennzeichnet, dass** die Verbindung, die das Präparat röntgenundurchlässig macht, ein inertes Oxid ist.

4. Präparat gemäß Anspruch 3, **dadurch gekennzeichnet, dass** die Verbindung, die das Präparat röntgenundurchlässig macht, ein Tantaloxid ist.

5. Präparat gemäß Anspruch 3, **dadurch gekennzeichnet, dass** die Verbindung, die das Präparat röntgenundurchlässig macht, ein Wolframoxid ist.

6. Präparat gemäß Anspruch 1, **dadurch gekennzeichnet, dass** das Ethanol eine Reinheit hat, die von 70 bis 99 Vol.-% variiert und vorzugsweise 95% beträgt.
